# EUROPÄISCHE PATENTSCHRIFT

(11) **EP 2 913 003 B1**
(45) Veröffentlichungstag und Bekanntmachung des Hinweises auf die Patenterteilung: **11.01.2023**
(21) Anmeldenummer: 14157369.1
(22) Anmeldetag: 28.02.2014
(51) Int. Cl.: A61B 5/097, A61B 5/024, A61B 5/083, A61B 5/087

(54) **Vorrichtung zur Entnahme einer Probe aus der menschlichen Atmung umfassend ein trichterförmiges und/oder rohrförmiges Gehäuseelement**
Device for taking a sample of human respiration comprising a flared and/or tubular housing element
Dispositif de prélèvement d'un échantillon à partir de la respiration humaine comprenant un élément de boîtier en forme d'entonnoir et/ou en forme de tube

(43) Veröffentlichungstag der Anmeldung: 02.09.2015
(73) Patentinhaber: pro4senses GmbH, 63571 Gelnhausen (DE)
(72) Erfinder: Jerichow, Ulrich, 63571 Gelnhausen (DE)
(74) Vertreter: Metten, Karl-Heinz

(56) Entgegenhaltungen:
- DE-A1- 4 127 599
- DE-B3-102005 020 102
- US-A1- 2002 122 746
- US-A1- 2004 186 390
- US-A1- 2007 068 810
- US-A1- 2013 236 980

## Beschreibung

Die Erfindung betrifft Vorrichtungen zur Entnahme einer Probe aus der menschlichen Atmung.

Dem Stand der Technik entsprechende Vorrichtungen zur Untersuchung der menschlichen Atmung werden zum Beispiel in DE4127599A1, US2004/0186390A1, US2002/0122746A1 und US2013/0236980A1 beschrieben.

Zur Steuerung und Überwachung eines Trainings von Personen ist es im Stand der Technik bekannt, eine maximale Sauerstoffaufnahme der Personen zu bestimmen. Dabei gilt die maximale Sauerstoffaufnahme (Vo₂ₘₐₓ) als klassischer Parameter zur Beurteilung der Ausdauerleistungsfähigkeit. Des Weiteren hat es sich zur Festlegung individuelle Trainingsintensitäten als vorteilhaft erwiesen zusätzlich den Parameter Vo₂ₘₐₓ zu erfassen. Für eine Bestimmung dieser Werte ist es erforderlich, die zu untersuchende Person maximal auszubelasten. Bestimmte Krankheitsbilder (z.B. Herzerkrankungen) schließen eine maximale Belastung aber von vornherein aus.

Im Stand der Technik sind daher bereits alternative Parameter zur Bestimmung der Leistungsfähigkeit einer Person bekannt. In der Leistungsdiagnostik wird die Leistungsfähigkeit einer Person oftmals aus respiratorischen Größen und/oder Laktatkonzentrationen im Blut bestimmt. Zum anderen erlauben Kinetiken der Herzfrequenz und der Sauerstoffaufnahme eine differenziertere Aussage über die limitierenden Faktoren der Vo₂ₘₐₓ.

Für die Erfassung der benötigten Messwerte sind im Stand der Technik Atemmasken oder Tuben bekannt, die Sensoren zur Bestimmung einer Sauerstoff- und Kohlendioxydkonzentration in der Inspirations- bzw. Expirationsluft und gegebenenfalls einen Volumenstrom der Inspirations- bzw. Expirationsluft eines Benutzers verwendet werden. Solche Atemmasken zeichnen sich dadurch aus, dass sie die Atemwege nach außen umschließen und von der Atemluft durchströmt werden. Dabei liegen die bekannten Atemmasken eng am Gesicht der zu testenden Personen an, oder werden im Falle eines Tubus vom Mund umschlossen, um diesen luftdicht abzuschließen.

Nachteilig an den bekannten Atemmasken oder Tuben ist, dass diese zu einem Wärmestau in der Maske und zu einem Kondensieren von Atemluft in der Maske führen, Abdrücke für längere Zeit auf dem Gesicht zurückbleiben, eine Engegefühl erzeugt wird und die zu testende Person gegen einen Luftwiderstand atmen muss, wodurch das Wohlbefinden des Trainierenden beeinflusst werden kann. Zudem sind die bekannten Atemmasken schwer und stören daher beim Training.

Die objektiv-technische Aufgabe der vorliegenden Erfindung ist daher, eine Vorrichtung bereitzustellen, die eine Messung der Sauerstoff- und/oder Kohlendioxydkonzentration in der Inspirations- bzw. Expirationsluft und gegebenenfalls einen Volumenstrom der Inspirations- bzw. Expirationsluft eines Benutzers ermöglicht, bei der das Wohlbefinden des Trainierenden, Anwendenden beim Atmen verbessert wird.

Die Aufgabe wird gelöst durch die Vorrichtung nach Anspruch 1.

Mit einer erfindungsgemäßen Vorrichtung kann ermöglicht werden, dass einzelne Atemzüge einer Person analysiert werden. Basierend auf dieser Analyse kann beispielsweise der respiratorische Quotient (Kohlenhydrat-/Fettverbrennung, Aerob/Anaerob) bestimmt werden. Auch kann mittels eines Belastungstests die maximale Sauerstoffaufnahme einer Person bestimmt werden. Zudem kann ermöglicht werden, eine Lungenventilation zu bestimmen. Selbstverständlich ist für einen Fachmann offensichtlich, dass weitere Anwendungen einer erfindungsgemäßen Vorrichtung möglich sind und diese nicht auf die exemplarisch aufgeführten voranstehenden Beispiele beschränkt sind.

Die erfindungsgemäße Vorrichtung hat insbesondere den Vorteil, dass das Gesicht des Trainierenden beim Ausführen einer Trainingseinheit im Wesentlichen nicht nach außen verschlossen ist, was zu einem angenehmeren Gefühl des Trainierenden im Zuge des Trainings führen kann. Zudem ist die erfindungsgemäße Vorrichtung leicht und erhöht daher den Tragekomfort zusätzlich. Weiterhin entstehen keine Druckstellen im Gesichtsbereich, der Schweiß kann einfach am Gesicht herunterlaufen und bleibt nicht in der Maske hängen, es muss nicht gegen einen Luftwiderstand geatmet werden und die Verwendung/Positionierung ist ohne Fachpersonal sicher möglich.

Mittels einer erfindungsgemäßen Vorrichtung können dabei beispielsweise alle Daten zur Bestimmung einer Leistungsfähigkeit einer Person ermittelt werden. Zudem können beispielsweise mittels einer erfindungsgemäßen Vorrichtung alle notwendigen Daten zur Steuerung und/oder Regelung der Trainings- und/oder Rehabilitationseinheit zur Verfügung gestellt werden, um beispielsweise Widerstands- und/oder Bremsanordnungen von Trainings- und/oder Rehabilitationseinheit entlang von Trainings-, Coaching- oder Rehabilitationszielen zu steuern oder zu regeln.

Ein erstes Halterungselement kann dabei erfindungsgemäß in unterschiedlichen Ausformungen bereitgestellt werden. Die Sensoreinheit kann erfindungsgemäß einteilig, jedoch auch in mindestens zwei oder mehr Teilen vorliegen, wobei jedes der Teile einen oder mehrere Sensoren umfassen kann.

Gemäß einer bevorzugten Ausführungsform der vorliegenden Erfindung kann es vorteilhaft sein, dass das mindestens eine trichterförmige und/oder rohrförmige Gehäuseelement ausgelegt und eingerichtet ist, um die Inspirations- und/oder Expirationsluft auf Basis eines vollständigen oder anteiligen Atemzugs des Benutzers teilweise oder vollständig an der mindestens einen Sensoreinheit vorbei, um die mindestens eine Sensoreinheit herum und/oder durch die Sensoreinheit hindurch zu leiten, wobei das mindestens eine trichterförmige und/oder rohrförmige Gehäuseelement insbesondere mindestens eine Aussparung aufweist, so dass die Inspirations- und/oder Expirationsluft des Benutzers, insbesondere zumindest, teilweise durch die Aussparung hindurch geleitet wird.

Durch das mindestens eine trichterförmige und/oder rohrförmige Gehäuseelement wird ein Luftleitelement für die Inspirations- und/oder Expirationsluft des Benutzers bereitgestellt.

Durch die erfindungsgemäße Halterung des trichterförmigen und/oder rohrförmigen Gehäuseelements kann ermöglicht werden, dass kein Wärmestau, keine Druckstellen und/oder keine Engegefühle in dem trichterförmigen und/oder rohrförmigen Gehäuseelement ermittelt und der Tragekomfort sowie Bedienkomfort für den Benutzer signifikant gesteigert wird.

Insbesondere kann es gemäß einer Ausführungsform der vorliegenden Erfindung vorteilhaft sein, dass das mindestens eine trichterförmige und/oder rohrförmige Gehäuseelement Kunststoff und/oder Poly(organo)siloxane umfasst oder aus Kunststoff und/oder Poly(organo)siloxanen besteht.

Poly(organo)siloxane (Silikon) haben sich als Material für das trichterförmige und/oder rohrförmige Gehäuseelement als vorteilhaft erwiesen. Es erfüllt die hygienischen anfordern und stellt ein kostengünstiges und leichtes Material für das trichterförmige und/oder rohrförmige Gehäuseelement bereit.

Dabei kann es gemäß einer Ausführungsform der vorliegenden Erfindung vorteilhaft sein, dass das mindestens eine erste Halterungselement mindestens einen Längsausleger umfasst, wobei das mindestens eine trichterförmige und/oder rohrförmige Gehäuseelement mittels des mindestens einen Längsauslegers und/oder mittels mindestens eines dritten Halterungselements auf mindestens einem zweiten Halterungspunkt am Kopf und/oder den Ohren des Benutzers halterbar ist oder gehaltert wird, wobei insbesondere das mindestens eine dritte Halterungselement in Form eines weiteren Längsauslegers ausgebildet ist und/oder das mindestens eine dritte Halterungselement an und/oder in dem mindestens einen Längsausleger, insbesondere entlang des mindestens einen Längsauslegers, beweglich gelagert ist.

Durch mindestens einen Längsausleger kann ein zweiter Halterungspunkt für die Vorrichtung am Kopf und/oder auf dem Ohr des Benutzers bereitgestellt werden. Mittels mindestens eines dritten Halterungselements, dass mit einem Längsausleger des ersten Halterungselements beweglich gelagert verbunden ist, kann zudem eine Anpassung an unterschiedliche Kopfformen und Größen erfolgen. Dabei ist der Längsausleger des ersten Halterungselements vorzugsweise in einem Winkel in einem Bereich von 70° bis 120°, insbesondere in einem Bereich von 80° bis 100°, in Bezug auf dem Querausleger, insbesondere an einem Ende des Querauslegers, angeordnet. Vorteilhaft kann gemäß einer Ausführungsform der Erfindung sein, dass insbesondere das dritte Halterungselement nahezu parallel zu dem mindestens einen Längsausleger des ersten Halterungselements, in dem mindestens einen ersten Längsausleger und/oder entlang des ersten Längsauslegers verschiebbar gelagert ist.

Die Kalibrierungseinheit ermöglicht den Verzicht auf eine externe Kalibrierung der Vorrichtung zur Erfassung exakter Messwerte.

Insbesondere kann es gemäß einer Ausführungsform der vorliegenden Erfindung vorteilhaft sein, dass das mindestens eine zweite Halterungselement mit dem mindestens einen Längsausleger und/oder dem mindestens einen Querausleger des mindestens einen ersten Halterungselements und mit der mindestens einen Sensoreinheit und/oder mit dem mindestens einen trichterförmigen und/oder rohrförmigen Gehäuseelement unmittelbar oder mittelbar verbindbar oder verbunden ist.

Auch kann es bevorzugt sein, dass das mindestens eine zweite Halterungselement am ersten Halterungspunkt und/oder zwischen einem ersten ersten Halterungspunkt und einem zweiten ersten Halterungspunkt, mit dem mindestens einen Querausleger und/oder an der dem zweiten Halterungspunkt gegenüberliegenden Seite des mindestens einen Längsauslegers mit dem mindestens einen Längsausleger mittelbar oder unmittelbar verbindbar oder verbunden ist.

Gemäß einer bevorzugten Ausführungsform der vorliegenden Erfindung kann es vorteilhaft sein, dass das mindestens eine zweite Halterungselement am ersten Halterungspunkt und/oder zwischen einem ersten Halterungspunkt und einem zweiten ersten Halterungspunkt, mit dem mindestens einen Querausleger und/oder an der dem zweiten Halterungspunkt gegenüberliegenden Seite des mindestens einen Längsauslegers mit dem mindestens einen Längsausleger mittelbar oder unmittelbar verbindbar oder verbunden ist.

Es kann durch ein Verbinden des mindestens einen zweiten Halterungselements mit dem mindestens einen Längsausleger und/oder dem mindestens einen Querausleger des mindestens einen ersten Halterungselements und mit der mindestens einen Sensoreinheit und/oder mit dem mindestens einen trichterförmigen und/oder rohrförmigen Gehäuseelement ermöglicht werden, dass das trichterförmige und/oder rohrförmige Gehäuseelement und die mindestens eine Sensoreinheit vor dem Mund und/oder der Nase eines Benutzers angeordnet werden, wobei die Vorrichtung selbst mittels des ersten und/oder dritten Halterungselements auf dem Kopf, insbesondere dem Hinterkopf, dem Nacken, der Nase und/oder den Ohren des Benutzers gehaltert ist. Dabei kann es gemäß einer Ausführungsform der vorliegenden Erfindung vorteilhaft sein, wenn das zweite Halterungselement mit dem mindestens einen Querausleger verbindbar oder verbunden ist. Die Verbindungsstelle von zweitem Halterungselement und Querausleger kann dabei beispielsweise im Bereich bzw. vor der Nase angeordnet sein. Gemäß einer weiteren Ausführungsform der vorliegenden Erfindung kann es vorteilhaft sein, wenn das zweite Halterungselement an dem mindestens einen Längsausleger angeordnet ist, so dass die mindestens eine Sensoreinheit und das mindestens eine trichterförmige Element seitlich von der Nase gehaltert ist.

Insbesondere kann es gemäß einer Ausführungsform der vorliegenden Erfindung vorteilhaft sein, dass das mindestens eine zweite Halterungselement an und/oder in dem mindestens einen Längsausleger, insbesondere entlang des mindestens einen Längsauslegers, und/oder an und/oder in dem mindestens einen Querausleger, insbesondere drehbar und/oder parallel entlang des mindestens einen Längsauslegers, beweglich gelagert ist.

Durch eine solche bewegliche Lagerung kann eine optimale Anpassung an verschiedene Kopfformen und Größen sichergestellt werden.

Gemäß einer bevorzugten Ausführungsform der vorliegenden Erfindung kann es vorteilhaft sein, dass das mindestens eine erste und/oder zweite Halterungselement mindestens eine Aufnahmeeinrichtung und/oder mindestens eine Halterungseinrichtung für das mindestens eine trichterförmige und/oder rohrförmige Gehäuseelement umfasst, und wobei die mindestens eine Aufnahmeeinrichtung und/oder die mindestens eine Halterungseinrichtung eine Nut und/oder Führung umfasst, in die das mindestens eine trichterförmige und/oder rohrförmige Gehäuseelement einführbar oder eingeführt ist, und wobei insbesondere ein Arretierelement, um das mindestens eine trichterförmige und/oder rohrförmige Gehäuseelement mit dem mindestens einen ersten und/oder zweiten Halterungselement zu verbinden umfasst ist, und wobei die mindestens eine Aufnahmeeinrichtung und/oder die die mindestens eine Führung und/oder die mindestens eine Nut mindestens einen Anschlag umfasst, um das Einführen des mindestens einen trichterförmigen und/oder rohrförmigen Gehäuseelements zu begrenzen und/oder wobei das mindestens eine trichterförmige Gehäuseelemente mit dem ersten und/oder zweiten Halterungselement mittels eines Klebemittels, insbesondere einem Klebstoff und/oder einem Pflaster, verbindbar oder verbunden ist.

Durch eine solche Halterungseinrichtung kann eine einfache und lösbare Verbindung zwischen dem mindestens einen trichterförmigen und/oder rohrförmigen Gehäuseelement und dem ersten und/oder zweiten Halterungselement bereitgestellt werden.

Gemäß einer bevorzugten Ausführungsform der vorliegenden Erfindung kann es vorteilhaft sein, dass das erste Halterungselement mindestens zwei Längsausleger umfasst, die gegenüberliegenden von dem mindestens einen ersten Halterungspunkt mit unmittelbar oder mittelbar mit dem mindestens einen Querausleger verbindbar oder verbunden sind und insbesondere zwei dritte Halterungselemente umfasst sind, so dass die Vorrichtung auf mindestens zwei zweiten Halterungspunkten am Kopf und/oder den Ohren des Benutzers halterbar ist oder gehaltert wird.

Durch mindestens zwei erste Längsausleger des ersten Halterungselements kann eine optimale Halterung der erfindungsgemäßen Vorrichtung, beispielsweise auf den Ohren eines Benutzers, ermöglicht werden. Durch zwei dritte Halterungselemente können die Sensoreinheit und das trichterförmige und/oder rohrförmige Gehäuseelement besonders sicher gehaltert werden.

Auch kann vorgesehen sein, dass mindestens zwei zweite Halterungselemente umfasst sind, wobei ein erstes zweites Halterungselement mit dem ersten Längsausleger des ersten Halterungselements und ein zweites zweites Halterungselement mit dem zweiten Längsausleger des ersten Halterungselements mittelbar oder unmittelbar verbindbar oder verbunden ist.

Mit dieser Anordnung ist eine besonders sichere Halterung der mindestens einen Sensoreinheit und des mindestens einen trichterförmigen und/oder rohrförmigen Gehäuseelements sowie eine variable Einstellung auf verschiedene Kopfgrößen und - formen ermöglicht.

Insbesondere kann es gemäß einer Ausführungsform der vorliegenden Erfindung vorteilhaft sein, dass das mindestens eine erste Halterungselement mittels mindestens eines elastischen Bands an dem Kopf eines Benutzers befestigbar oder befestigt ist und/oder aus diesem gebildet ist.

Insbesondere kann es gemäß einer Ausführungsform der vorliegenden Erfindung vorteilhaft sein, dass die Vorrichtung mindestens ein Mikrofon und/oder mindestens einen Lautsprecher, insbesondere eine Sprechgarnitur, insbesondere ein Bluetooth Headset, umfass. Mittels des mindestens einen Mikrofons und/oder des mindestens einen Lautsprechers kann eine Kommunikation mit der Person während eines Trainings ermöglicht werden.

Auch kann gemäß einer bevorzugten Ausführungsform der vorliegenden Erfindung vorgesehen sein, dass eine Datenverarbeitungseinrichtung umfasst ist, die mit dem der Sensoreinheit, dem Mikrofon und/oder dem Lautsprecher, insbesondere der Sprechgarnitur, in Wirkverbindung steht und die insbesondere für einen drahtlosen Datenaustausch der Sensoreinheit, des Mikrofons und/oder des Lautsprechers, insbesondere der Sprechgarnitur, mit einer weiteren Datenverarbeitungseinrichtung ausgelegt und eingerichtet ist, insbesondere über Bluetooth, Infrarot und/oder Wireless-Lan.

Ein solcher drahtloser Datenaustausch, insbesondere in Kombination mit einem von der erfindungsgemäßen Vorrichtung umfassten Akkumulator, kann vorteilhaft sein, um eine bei Belastungstest möglicherweise störende Datenkabel zu vermeiden. Datenkabel können das Risiko beinhaltet, dass diese in Elementen von Trainings- und Rehabilitationseinrichtungen verhaken, und sind zudem bei freiem Training nachteilig, da der Bewegungsradius des Benutzers durch die Kabellänge begrenzt ist.

Gemäß einer bevorzugten Ausführungsform der vorliegenden Erfindung kann es vorteilhaft sein, dass das mindestens eine erste, das mindestens eine zweite und/oder das mindestens eine dritte Halterungselement aus Kunststoff und/oder Silikon besteht oder Kunststoff und/oder Silikon umfasst, insbesondere aus transparentem Kunststoff.

Durch diese Materialien wird eine besonders leichte Ausführung der erfindungsgemäßen Vorrichtung ermöglicht um einen besonders hohen Tragekomfort bereitzustellen.

Auch kann es vorgesehen sein, dass der mindestens eine vierte Sensor zum Bestimmen einer Herzfrequenz des Benutzers in der mindestens einen Sensoreinheit oder getrennt von dieser anordnenbar oder angeordnet ist, insbesondere am Körper des Benutzers anordnenbar oder angeordnet ist, wobei der mindestens eine vierte Sensor insbesondere in Form eines, insbesondere kabellosen, Herzfrequenzmessgerät und/oder eines Sauerstoffplusmesser ausgebildet ist.

Herzfrequenzmessgeräte sind im Stand der Technik bekannt. Diese messen die Anzahl der Herzschläge pro Minute. Herzfrequenzmessgeräte können dabei in Form einer sogenannten Pulsuhr oder an einem Brustband befestigt werden. Andere Herzfrequenzmessgeräte messen die Herzfrequenz am Ohrläppchen. Auch ist eine Sauerstoffplusmessung im Stand der Technik bekannt.

Gemäß einer bevorzugten Ausführungsform der vorliegenden Erfindung kann es vorteilhaft sein, dass die mindestens eine Sensoreinheit getrennt von dem mindestens einen trichterförmigen Element vorliegt oder die mindestens eine Sensoreinheit und das mindestens einen trichterförmigen Element lösbar miteinander verbindbar oder verbunden sind.

Dies ermöglicht einen besonders einfachen Austausch des trichterförmigen und/oder rohrförmigen Gehäuseelements.

Auch liefert die Erfindung ein trichterförmiges und/oder rohrförmiges Gehäuseelement, wobei das trichterförmige und/oder rohrförmige Gehäuseelement mit mindestens einer Sensoreinheit in Wirkverbindung bringbar ist oder steht, und wobei die mindestens eine Sensoreinheit ausgelegt und eingerichtet ist, um eine Sauerstoff- und/oder Kohlendioxydkonzentration in der Inspirations- und/oder Expirationsluft und/oder ein Volumenstrom der Inspirations- und/oder Expirationsluft des Benutzers zu messen, und das trichterförmige und/oder rohrförmige Gehäuseelement ausgelegt und eingerichtet ist, um die Inspirations- und/oder Expirationsluft eines Benutzers teilweise oder vollständig an der mindestens einen Sensoreinheit vorbei, um die mindestens eine Sensoreinheit herum und/oder durch die Sensoreinheit hindurch zu leiten und wobei das trichterförmige und/oder rohrförmige Gehäuseelement aus Silikon besteht oder umfasst.

Auch liefert die Erfindung eine Verwendung eine erfindungsgemäßen Vorrichtung für eine Atemgasanalyse.

Schließlich liefert die Erfindung Verwendung einer erfindungsgemäßen Vorrichtung zur Erfassung von Messdaten zur Steuerung oder Regelung mindestens einer steuerbaren oder regelbaren Widerstandseinrichtung einer Trainings- und/oder Rehabilitationseinheit.

Der Erfindung liegt die überraschende Erkenntnis zu Grunde, dass mittels mindestens eines ersten Halterungselements, mindestens einer Sensoreinheit und mindestens eines trichterförmigen und/oder rohrförmigen Gehäuseelements ein leichte Vorrichtung bereitgestellt werden kann, die einen hohen Tragekomfort während sportlichen Aktivitäten ermöglicht, wobei gleichzeitig eine Analyse der Atemgase stattfinden kann.

Weitere Merkmale und Vorteile der Erfindung ergeben sich aus der nachfolgenden Beschreibung, in der Ausführungsbeispiele der Erfindung anhand von schematischen Zeichnungen beispielhaft erläutert werden, ohne dadurch die Erfindung zu beschränken. Dabei zeigt:
- Figur 1a:: eine schematische perspektivische Ansicht einer ersten Ausführungsform einer erfindungsgemäßen Vorrichtung;
- Figur 1b:: eine schematische Ansicht teilweise im Schnitt der ersten Ausführungsform einer erfindungsgemäßen Vorrichtung;
- Figur 2a:: eine schematische perspektivische Ansicht einer zweiten Ausführungsform einer erfindungsgemäßen Vorrichtung;
- Figur 2b:: eine schematische seitliche Ansicht der zweiten Ausführungsform einer erfindungsgemäßen Vorrichtung;
- Figur 3a:: eine schematische perspektivische Ansicht einer dritten Ausführungsform einer erfindungsgemäßen Vorrichtung; und
- Figur 3b:: eine schematische seitliche Ansicht der dritten Ausführungsform einer erfindungsgemäßen Vorrichtung;

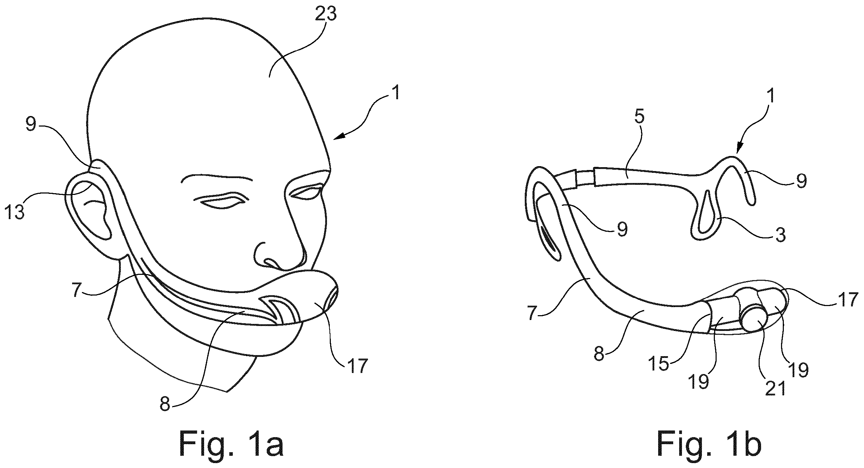

In den Figuren 1a und 1b ist eine erste Ausführungsform einer erfindungsgemäßen Vorrichtung 1 gezeigt. Die Vorrichtung 1 umfasst ein erstes Halterungselement 3 mit einem Querausleger 5 und einem Längsausleger 7. Des weiteren umfasst die Vorrichtung 1 zwei zweite Halterungselemente 9, die mit den ersten Halterungselementen 3 verbunden sind. Mittels des ersten Haltungselements 3 ist die Vorrichtung 1 auf ersten Halterungspunkten im Nacken bzw. auf dem Hinterkopf eines Benutzers gehaltert, mittels der zweiten Halterungselemente 9 auf zwei zweiten Halterungspunkten 13 auf den Ohren des Benutzers.

Mittels eines sich an den Längsausleger 7 anschließenden weiteren Querauslegers 8 bzw. eines dritten Halterungselements 15 ist ein trichterförmiges und/oder rohrförmiges Gehäuseelement 17 und eine Sensoreinheit 19 an dem ersten Halterungselement 3 gehaltert. Die erste Sensoreinheit 19 ist dabei zweitteilig ausgeführt. Über eine Aussparung 21 in Form einer Trichteröffnung kann die Inspirations- und/oder Expirationsluft eines Benutzers durch das trichterförmige und/oder rohrförmige Gehäuseelement 17 geführt werden. Wir aus den Figuren 1a und 1b ersichtlich, liegt die Vorrichtung 1 nicht eng an dem Kopf eines Benutzers 23 an, sondern stellt einen nicht abgeschlossenen Volumenraum bereit.

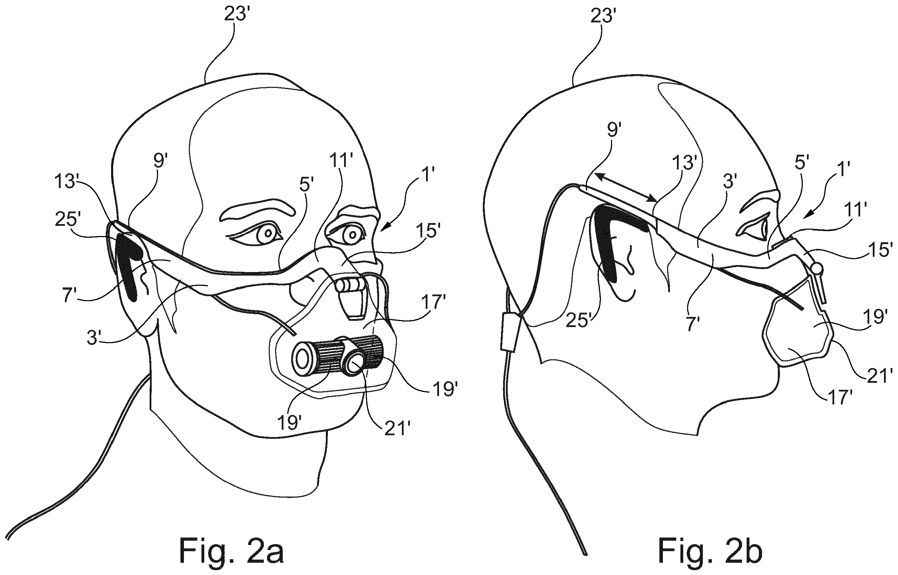

In den Figuren 2a und 2b ist eine zweite Ausführungsform einer erfindungsgemäßen Vorrichtung 1' gezeigt. Die Vorrichtung 1 umfasst ein erstes Halterungselement 3' mit einem Querausleger 5' und zwei Längsauslegem 7', wobei nur einer der beiden Längsausleger 7' gezeigt ist. Des weiteren umfasst die Vorrichtung 1' zwei zweite Halterungselemente 9', die zur Anpassung der Vorrichtung an unterschiedliche Kopfformen und -größen beweglich mit den ersten Halterungselementen 3' verbunden sind. Dabei ist nur ein zweites Halterungselement 9' gezeigt. Mittels des ersten Haltungselements 3' ist die Vorrichtung 1' auf ersten Halterungspunkten 11' auf der Nase eines Benutzers gehaltert, mittels der zweiten Halterungselemente 9' auf zwei zweiten Halterungspunkten 13' auf den Ohren des Benutzers.

Mittels eines dritten Halterungselements 15' ist ein trichterförmiges und/oder rohrförmiges Gehäuseelement 17' und eine Sensoreinheit 19' an dem ersten Halterungselement 3' gehaltert. Die erste Sensoreinheit 19' ist dabei zweitteilig ausgeführt. Über eine Aussparung 21' in Form einer Trichteröffnung kann die Inspirations- und/oder Expirationsluft eines Benutzers durch das trichterförmige und/oder rohrförmige Gehäuseelement 17' geführt werden. Wir aus den Figuren 2a und 2b ersichtlich, liegt die Vorrichtung 1 nicht eng an dem Kopf eines Benutzers 23 an, sondern stellt einen nicht abgeschlossenen Volumenraum bereit.

Des weiteren umfasst die erfindungsgemäße Vorrichtung 1 einen Lautsprecher 25' sowie ein Mirkofon (nicht gezeigt), die insbesondere eine Kommunikation mit dem Benutzer über Bluetooth ermöglichen.

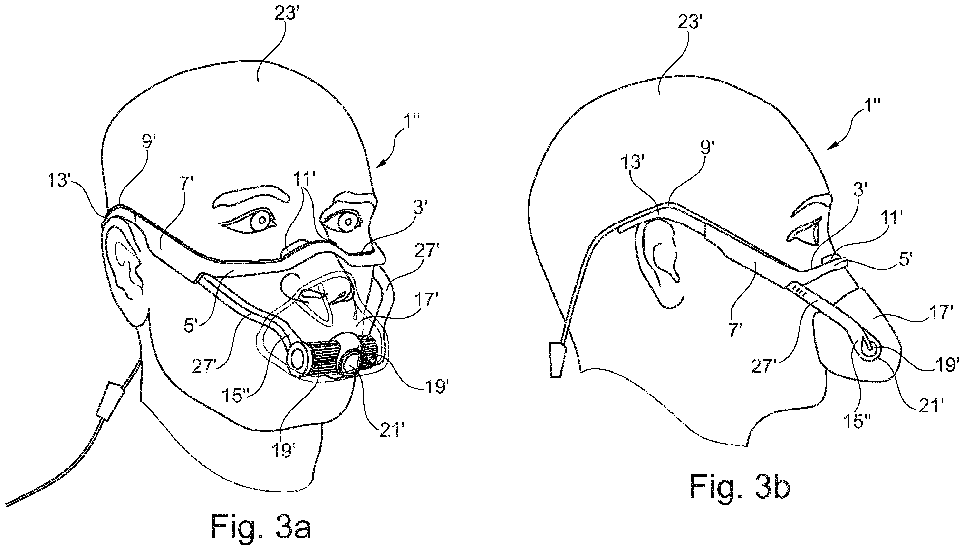

In den Figuren 3a und 3b ist eine dritte alternative Ausführungsform einer erfindungsgemäßen Vorrichtung 1" gezeigt. Diese unterscheidet sich von der Vorrichtungen 1 und 1' gemäß den Figuren la, 1b, 2a, und 2b durch die Ausgestaltung des dritten Halterungselements 15". Das dritte Halterungselement 15" umfasst dabei zwei weitere Längsausleger 27', die mit dem trichterförmigen und/oder rohrförmigen Gehäuseelement 17' und mit der Sensoreinheit 19' verbunden sind.

Die Erfindung ist in den Ansprüchen definiert.

## Patentansprüche

1. Vorrichtung (1, 1') zur Entnahme einer Probe aus der menschlichen Atmung eines Trainierenden, umfassend mindestens ein erstes Halterungselement (3, 3'), mindestens eine Sensoreinheit (19, 19') umfassend eine Kalibrierungseinrichtung sowie mindestens einen ersten Sensor zur Bestimmung einer Sauerstoffkonzentration einer Inspirations- und/oder Expirationsluft eines Benutzers und/oder mindestens einen zweiten Sensor zur Bestimmung einer Kohlendioxydkonzentration der Inspirations- und/oder Expirationsluft des Benutzers und/oder mindestens einen dritten Sensor zur Bestimmung des Volumenstroms der Inspirations- und/oder Expirationsluft des Benutzers und optional mindestens einen vierten Sensor zum Bestimmen einer Herzfrequenz des Benutzers, und
mindestens ein trichterförmiges und/oder rohrförmiges Gehäuseelement (17, 17') wobei das mindestens eine erste Halterungselement mindestens einen Querausleger (5, 5') umfasst,
wobei der mindestens eine Querausleger ausgelegt und eingerichtet ist, um die Vorrichtung auf mindestens einem ersten Halterungspunkt (13, 13'), insbesondere auf einer Nase, dem Hinterkopf und/oder dem Nacken eines Benutzers, zu halten, und
das mindestens eine erste Halterungselement mit dem mindestens einen trichterförmigen und/oder rohrförmigen Gehäuseelement unmittelbar oder mittelbar, insbesondere über mindestens ein zweites Halterungselement (9, 9'), verbindbar oder verbunden ist,
so dass das mindestens eine trichterförmige und/oder rohrförmige Gehäuseelement vor einem Mund des Benutzers halterbar ist oder gehaltert wird, und
wobei die mindestens eine Sensoreinheit mit dem mindestens einen ersten Halterungselement, dem mindestens einen zweiten Halterungselement und/oder dem mindestens einen trichterförmigen und/oder rohrförmigen Gehäuseelement verbindbar oder verbunden ist,
wobei die mindestens eine Sensoreinheit innerhalb des mindestens einen trichterförmigen und/oder rohrförmigen Gehäuseelements angeordnet ist,
wobei die Sensoreinheit ausgelegt und eingerichtet ist, eine Sauerstoff- und/oder Kohlendioxydkonzentration in der Inspirations- und/oder Expirationsluft und/oder einen Volumenstrom der Inspirations- und/oder Expirationsluft und optional eine Herzfrequenz des Benutzers zu messen,
während das mindestens eine trichterförmige und/oder rohrförmige Gehäuseelement beabstandet vor dem Mund des Benutzers gehaltert ist, so dass durch das trichterförmige und/oder rohrförmige Gehäuseelement ein teilweise offener Volumenraum für die Inspirations- und/oder Expirationsluft bereitgestellt wird,
so dass das Gesicht des Trainierenden im Wesentlichen nicht nach außen verschlossen ist.

2. Vorrichtung nach Anspruch 1, **dadurch gekennzeichnet, dass**
das mindestens eine trichterförmige und/oder rohrförmige Gehäuseelement ausgelegt und eingerichtet ist, um die Inspirations- und/oder Expirationsluft auf Basis eines vollständigen oder anteiligen Atemzugs des Benutzers teilweise oder vollständig an der mindestens einen Sensoreinheit vorbei, um die mindestens eine Sensoreinheit herum und/oder durch die Sensoreinheit hindurch zu leiten, wobei das mindestens eine trichterförmige und/oder rohrförmige Gehäuseelement insbesondere mindestens eine Aussparung aufweist, so dass die Inspirations- und/oder Expirationsluft des Benutzers, insbesondere zumindest, teilweise durch die Aussparung hindurch geleitet wird.

3. Vorrichtung nach Anspruch 1 oder 2, **dadurch gekennzeichnet, dass**
das mindestens eine erste Halterungselement mindestens einen Längsausleger umfasst, wobei das mindestens eine trichterförmige und/oder rohrförmige Gehäuseelement mittels des mindestens einen Längsauslegers und/oder mittels mindestens eines dritten Halterungselements auf mindestens einem zweiten Halterungspunkt am Kopf und/oder den Ohren des Benutzers halterbar ist oder gehaltert wird, wobei insbesondere das mindestens eine dritte Halterungselement in Form eines weiteren Längsauslegers ausgebildet ist und/oder das mindestens eine dritte Halterungselement an und/oder in dem mindestens einen Längsausleger, insbesondere entlang des mindestens einen Längsauslegers, beweglich gelagert ist.

4. Vorrichtung nach einem der vorangehenden Ansprüche, **dadurch gekennzeichnet, dass**
das mindestens eine zweite Halterungselement, mit dem mindestens einen Längsausleger und/oder dem mindestens einen Querausleger des mindestens einen ersten Halterungselements und mit der mindestens einen Sensoreinheit und/oder mit dem mindestens einen trichterförmigen und/oder rohrförmigen Gehäuseelement unmittelbar oder mittelbar verbindbar oder verbunden ist und/oder das mindestens eine zweite Halterungselement am ersten Halterungspunkt und/oder zwischen einem ersten ersten Halterungspunkt und einem zweiten ersten Halterungspunkt, mit dem mindestens einen Querausleger und/oder an der dem zweiten Halterungspunkt gegenüberliegenden Seite des mindestens einen Längsauslegers mit dem mindestens einen Längsausleger mittelbar oder unmittelbar verbindbar oder verbunden ist.

5. Vorrichtung nach einem der vorangehenden Ansprüche, **dadurch gekennzeichnet, dass**
das mindestens eine erste und/oder zweite Halterungselement mindestens eine Aufnahmeeinrichtung und/oder mindestens eine Halterungseinrichtung für das mindestens eine trichterförmige und/oder rohrförmige Gehäuseelement umfasst, und wobei die mindestens eine Aufnahmeeinrichtung und/oder die mindestens eine Halterungseinrichtung eine Nut und/oder Führung umfasst, in die das mindestens eine trichterförmige und/oder rohrförmige Gehäuseelement einführbar oder eingeführt ist, und wobei insbesondere ein Arretierelement, um das mindestens eine trichterförmige und/oder rohrförmige Gehäuseelement mit dem mindestens einen ersten und/oder zweiten Halterungselement zu verbinden umfasst ist, und wobei die mindestens eine Aufnahmeeinrichtung und/oder die die mindestens eine Führung und/oder die mindestens eine Nut mindestens einen Anschlag umfasst, um das Einführen des mindestens einen trichterförmigen und/oder rohrförmigen Gehäuseelements zu begrenzen und/oder wobei das mindestens eine trichterförmige Gehäuseelemente mit dem ersten und/oder zweiten Halterungselement mittels eines Klebemittels, insbesondere einem Klebstoff und/oder einem Pflaster, verbindbar oder verbunden ist.

6. Vorrichtung nach einem der vorangehenden Ansprüche, **dadurch gekennzeichnet, dass**
das erste Halterungselement mindestens zwei Längsausleger umfasst, die gegenüberliegend von dem mindestens einen ersten Halterungspunkt unmittelbar oder
mittelbar mit dem mindestens einen Querausleger verbindbar oder verbunden sind und insbesondere zwei dritte Halterungselemente umfasst sind, so dass die Vorrichtung auf mindestens zwei zweiten Halterungspunkten am Kopf und/oder den Ohren des Benutzers halterbar ist oder gehaltert wird.

7. Vorrichtung nach einem der vorangehenden Ansprüche, **dadurch gekennzeichnet, dass**
das mindestens eine erste Halterungselement mittels mindestens eines elastischen Bands an dem Kopf eines Benutzers befestigbar oder befestigt ist und/oder aus diesem gebildet ist.

8. Vorrichtung nach einem der vorangehenden Ansprüche, **dadurch gekennzeichnet, dass**
die Vorrichtung mindestens ein Mikrofon und/oder mindestens einen Lautsprecher, insbesondere eine Sprechgarnitur, insbesondere ein Bluetooth Headset, umfasst.

9. Vorrichtung nach einem der vorangehenden Ansprüche, **dadurch gekennzeichnet, dass**
eine Datenverarbeitungseinrichtung umfasst ist, die mit der Sensoreinheit, dem Mikrofon und/oder dem Lautsprecher, insbesondere der Sprechgarnitur, in Wirkverbindung steht und die insbesondere für einen drahtlosen Datenaustausch der Sensoreinheit, des Mikrofons und/oder des Lautsprechers, insbesondere der Sprechgarnitur, mit einer weiteren Datenverarbeitungseinrichtung ausgelegt und eingerichtet ist, insbesondere über Bluetooth, Infrarot und/oder Wireless-Lan.

10. Vorrichtung nach einem der vorangehenden Ansprüche, **dadurch gekennzeichnet, dass**
die mindestens eine Sensoreinheit getrennt von dem mindestens einen trichterförmigen Element vorliegt oder die mindestens eine Sensoreinheit und das mindestens eine trichterförmige Element lösbar miteinander verbindbar oder verbunden sind.

11. Vorrichtung nach einem der vorangehenden Ansprüche, **dadurch gekennzeichnet, dass**
der mindestens eine vierte Sensor zum Bestimmen einer Herzfrequenz des Benutzers in der mindestens einen Sensoreinheit oder getrennt von dieser anordnenbar oder angeordnet ist, insbesondere am Körper des Benutzers anordnenbar oder angeordnet ist, wobei der mindestens eine vierte Sensor insbesondere in Form eines, insbesondere kabellosen, Herzfrequenzmessgerät und/oder eines Sauerstoffplusmesser ausgebildet ist.

12. Verwendung eine Vorrichtung nach einem der Ansprüche 1 bis 11 für eine Atemgasanalyse und/oder zur Erfassung von Messdaten zur Steuerung oder Regelung mindestens einer steuerbaren oder regelbaren Widerstandseinrichtung einer Trainings- und/oder Rehabilitationseinheit.

## Claims

1. A device (1, 1') for taking a sample of human respiration of a person exercising, comprising
at least one first holding element (3, 3'),
at least one sensor unit (19, 19') comprising a calibration component as well as at least one first sensor for determining an oxygen concentration of inspired and/or expired air of a user, and/or
at least one second sensor for determining a carbon dioxide concentration of the inspired and/or expired air of the user, and/or
at least one third sensor for determining the volume flow of the inspired and/or expired air of the user and, optionally,
at least one fourth sensor for determining a heart rate of the user, and
at least one funnel-shaped and/or tubular housing element (17, 17'),
wherein the at least one first holding element comprises at least one transverse arm (5, 5'),
wherein the at least one transverse arm is adapted and arranged to hold the device on at least one first holding point (13, 13'), in particular on a nose, the back of the head and/or the back of the neck of a user, and
the at least one first holding element can be or is connected directly or indirectly, in particular via at least one second holding element (9, 9'), to the at least one funnel-shaped and/or tubular housing element,
such that the at least one funnel-shaped and/or tubular housing element can be or is held in front of a mouth of the user, and
wherein the at least one sensor unit can be or is connected to the at least one first holding element, the at least one second holding element and/or the at least one funnel-shaped and/or tubular housing element,
wherein the at least one sensor unit is arranged inside of the at least one funnel-shaped and/or tubular housing element, wherein the sensor unit is adapted and arranged to measure an oxygen and/or carbon dioxide concentration in the inspired and/or expired air and/or a volume flow of the inspired and/or expired air and, optionally, to measure a heart rate of the user, while the at least one funnel-shaped and/or tubular housing element is held at a distance in front of the mouth of the user, such that a partially open volumetric space for the inspired and/or expired air is provided by the funnel-shaped and/or tubular housing element,
such that the face of the person exercising is substantially not sealed to the outside.

2. The device according to Claim 1, **characterized in that**
the at least one funnel-shaped and/or tubular housing element is adapted and arranged to guide the inspired and/or expired air on the basis of a complete or partial breath of the user partially or completely past the at least one sensor unit, around the at least one sensor unit and/or through the sensor unit, wherein the at least one funnel-shaped and/or tubular housing element has in particular at least one recess such that the inspired and/or expired air of the user is guided, in particular at least, partially through the recess.

3. The device according to Claim 1 or 2, **characterized in that**
the at least one first holding element comprises at least one longitudinal arm, wherein the at least one funnel-shaped and/or tubular housing element can be or is held on at least one second holding point on the head and/or the ears of the user by means of the at least one longitudinal arm and/or by means of at least one third holding element, wherein the at least one third holding element is in particular configured in the form of a further longitudinal arm and/or the at least one third holding element is movably supported on and/or in the at least one longitudinal arm, in particular along the at least one longitudinal arm.

4. The device according to any one of the preceding claims, **characterized in that**
the at least one second holding element can be or is connected directly or indirectly to the at least one longitudinal arm and/or the at least one transverse arm of the at least one first holding element and to the at least one sensor unit and/or to the at least one funnel-shaped and/or tubular housing element, and/or
the at least one second holding element can be or is connected directly or indirectly to the at least one transverse arm at the first holding point and/or between a first first holding point and a second first holding point, and/or to the at least one longitudinal arm at the side of the at least one longitudinal arm opposite the second holding point.

5. The device according to any one of the preceding claims, **characterized in that**
the at least one first and/or second holding element comprises at least one receiving component and/or at least one holding component for the at least one funnel-shaped and/or tubular housing element, and wherein the at least one receiving component and/or the at least one holding component comprises a groove and/or guide, into which the at least one funnel-shaped and/or tubular housing element can be or is introduced, and wherein in particular an locking element is comprised in order to connect the at least one funnel-shaped and/or tubular housing element to the at least one first and/or second holding element, and wherein the at least one receiving component and/or the at least one guide and/or the at least one groove comprises at least one stopper in order to limit the introduction of the at least one funnel-shaped and/or tubular housing element, and/or wherein the at least one funnel-shaped housing element can be or is connected to the first and/or second holding element by means of an adhesive means, in particular an adhesive and/or a plaster.

6. The device according to any one of the preceding claims, **characterized in that**
the first holding element comprises at least two longitudinal arms which can be or are connected directly or indirectly to the at least one transverse arm opposite the at least one first holding point and, in particular, two third holding elements are comprised such that the device can be or is held on at least two second holding points on the head and/or the ears of the user.

7. The device according to any one of the preceding claims, **characterized in that**
the at least one first holding element can be or is fastened to the head of a user by means of at least one elastic band and/or is formed from said elastic band.

8. The device according to any one of the preceding claims, **characterized in that** the device comprises at least one microphone and/or at least one loudspeaker, in particular a headset, in particular a Bluetooth headset.

9. The device according to any one of the preceding claims, **characterized in that**
a data processing component is comprised, which is in operative connection with the sensor unit, the microphone and/or the loudspeaker, in particular the headset, and which is in particular adapted and arranged for a wireless data exchange of the sensor unit, the microphone and/or the loudspeaker, in particular the headset, with a further data processing component, in particular via Bluetooth, infrared and/or wireless LAN.

10. The device according to any one of the preceding claims, **characterized in that**
the at least one sensor unit is separated from the at least one funnel-shaped element, or the at least one sensor unit and the at least one funnel-shaped element can be or are detachably connected to one another.

11. The device according to any one of the preceding claims, **characterized in that**
the at least one fourth sensor for determining a heart rate of the user can be or is arranged in the at least one sensor unit or separately from the latter, in particular can be or is arranged on the body of the user, wherein the at least one fourth sensor is in particular configured in the form of a, in particular wireless, heart rate measuring appliance and/or an oxygen pulse meter.

12. Use of a device according to any one of Claims 1 to 11 for a breathing gas analysis and/or for capturing measuring data for controlling or regulating at least one controllable or regulatable resistance component of a training and/or rehabilitation unit.

## Revendications

1. Dispositif (1, 1') pour le prélèvement d'un échantillon de respiration humaine d'une personne en entraînement, comprenant
au moins un premier élément de maintien (3, 3'),
au moins une unité de capteur (19, 19') comprenant une pièce de calibration ainsi qu'au moins un premier capteur pour la détermination de la concentration en oxygène de l'air d'inspiration et/ou d'expiration d'un utilisateur et/ou
au moins un deuxième capteur pour la détermination de la concentration en dioxyde de carbone de l'air d'inspiration et/ou d'expiration de l'utilisateur et/ou
au moins un troisième capteur pour la détermination du débit volumique de l'air d'inspiration et/ou d'expiration de l'utilisateur, et facultativement
au moins un quatrième capteur pour la détermination de la fréquence cardiaque de l'utilisateur, et
au moins un élément de boîtier (17, 17') en forme d'entonnoir et/ou de tube,
ledit au moins un premier élément de maintien comprenant au moins un bras transversal (5, 5'),
ledit au moins un bras transversal étant conçu et adapté pour maintenir le dispositif sur au moins un premier point de retenue (13, 13'), en particulier sur le nez, l'occiput et/ou la nuque d'un utilisateur, et
ledit au moins un premier élément de maintien pouvant être ou étant connecté, directement ou indirectement, audit au moins un élément de boîtier en forme d'entonnoir et/ou de tube, en particulier par au moins un deuxième élément de maintien (9, 9'),
si bien que ledit au moins un élément de boîtier en forme d'entonnoir et/ou de tube peut être maintenu ou est maintenu devant une bouche de l'utilisateur, et
ladite au moins une unité de capteur pouvant être ou étant connecté audit au moins un premier élément de maintien, audit au moins un deuxième élément de maintien et/ou audit au moins un élément de boîtier en forme d'entonnoir et/ou de tube,
ladite au moins une unité de capteur étant disposée à l'intérieur dudit au moins un élément de boîtier en forme d'entonnoir et/ou de tube, l'unité de capteur étant conçue et adaptée pour mesurer une concentration en oxygène et/ou en dioxyde de carbone dans l'air d'inspiration et/ou d'expiration et/ou un débit volumique de l'air d'inspiration et/ou d'expiration et facultativement une fréquence cardiaque de l'utilisateur, pendant que ledit au moins un élément de boîtier en forme d'entonnoir et/ou de tube est maintenu espacé avant de la bouche de l'utilisateur,
si bien que par ledit au moins un élément de boîtier en forme d'entonnoir et/ou de tube, un espace volumique partiellement ouvert est mis à disposition pour l'air d'inspiration et/ou d'expiration,
de sorte que le visage de la personne en entraînement n'est sensiblement pas fermé vers t'extérieur.

2. Dispositif selon la revendication 1, **caractérisé en ce que**
ledit au moins un élément de boîtier en forme d'entonnoir et/ou de tube est conçu et adapté pour faire passer l'air d'inspiration et/ou d'expiration sur la base d'un souffle complet ou partiel de l'utilisateur partiellement ou totalement par ladite au moins une unité de capteur, en entourant ladite au moins une unité de capteur et/ou en traversant l'unité de capteur, ledit au moins un élément de boîtier en forme d'entonnoir et/ou de tube présentant en particulier au moins une cavité, de sorte que l'air d'inspiration et/ou d'expiration de l'utilisateur est, en particulier au moins, partiellement conduit en traversant la cavité.

3. Dispositif selon la revendication 1 ou 2, **caractérisé en ce que**
ledit au moins un premier élément de maintien comprend au moins un bras longitudinal, ledit au moins un élément de boîtier en forme d'entonnoir et/ou de tube pouvant être ou étant retenu au moyen dudit au moins un bras longitudinal et/ou au moyen d'au moins un troisième élément de maintien sur au moins un deuxième point de retenue contre la tête et/ou les oreilles de l'utilisateur, ledit au moins un troisième élément de maintien étant prévu en particulier sous la forme d'un autre bras longitudinal et/ou ledit au moins un troisième élément de maintien étant monté de manière mobile contre et/ou dans ledit au moins un bras longitudinal, en particulier le long dudit au moins un bras longitudinal.

4. Dispositif selon l'une quelconque des revendications précédentes, **caractérisé en ce que**
ledit au moins un deuxième élément de maintien peut être ou est connecté, directement ou indirectement, audit au moins un bras longitudinal et/ou audit au moins un bras transversal dudit au moins un premier élément de maintien et à ladite au moins une unité de capteur et/ou audit au moins un élément de boîtier en forme d'entonnoir et/ou de tube, et/ou
ledit au moins un deuxième élément de maintien, sur le premier point de retenue et/ou entre un premier premier point de retenue et un deuxième premier point de retenue, peut être ou est connecté, directement ou indirectement, audit au moins un bras transversal et/ou audit au moins un bras longitudinal sur le côté dudit au moins un bras longitudinal opposé au deuxième point de retenue.

5. Dispositif selon l'une quelconque des revendications précédentes, **caractérisé en ce que**
ledit au moins un premier et/ou deuxième élément de maintien comprend au moins une pièce de réception et/ou au moins une pièce de maintien pour ledit au moins un élément de boîtier en forme d'entonnoir et/ou de tube, et où ledit au moins une pièce de réception et/ou ledit au moins une pièce de maintien présente une rainure et/ou un guidage, où ledit au moins un élément de boîtier en forme d'entonnoir et/ou de tube peut être ou est inséré, et où est compris en particulier un élément d'arrêt, pour connecter ledit au moins un élément de boîtier en forme d'entonnoir et/ou de tube audit au moins un premier et/ou un deuxième élément de maintien, et où ledit au moins une pièce de réception et/ou ledit au moins un guidage et/ou ladite au moins une rainure comprend au moins une butée, destinée à limiter l'insertion dudit au moins un élément de boîtier en forme d'entonnoir et/ou de tube, et/ou ledit au moins un élément de boîtier en forme d'entonnoir peut être ou est connecté au premier et/ou deuxième élément de maintien au moyen d'un moyen adhésif, en particulier d'une colle et/ou d'un sparadrap.

6. Dispositif selon l'une quelconque des revendications précédentes, **caractérisé en ce que**
le premier élément de maintien comprend au moins deux bras longitudinaux, lesquels peuvent être ou sont connectés, directement ou indirectement, audit au moins un bras transversal à l'opposé dudit au moins un premier point de retenue, et où en particulier deux troisièmes éléments de maintien sont compris, si bien que le dispositif peut être ou est maintenu contre la tête et/ou les oreilles de l'utilisateur sur au moins deux deuxièmes points de retenue.

7. Dispositif selon l'une quelconque des revendications précédentes, **caractérisé en ce que**
ledit au moins un premier élément de maintien peut être ou est fixé contre la tête d'un utilisateur au moyen d'au moins une bande élastique et/ou est formé par celle-ci.

8. Dispositif selon l'une quelconque des revendications précédentes, **caractérisé en ce que**
ledit dispositif comprend au moins un microphone et/ou au moins un haut-parieur, en particulier un casque, en particulier un casque Bluetooth.

9. Dispositif selon l'une quelconque des revendications précédentes, **caractérisé en ce qu'**une pièce de traitement des données est comprise, lequel se trouve en liaison fonctionnelle avec l'unité de capteur, le microphone et/ou le haut-parleur, en particulier le casque, et est en particulier conçue et adapté pour une communication sans fil de données de l'unité de capteur, du microphone et/ou du haut-parleur, en particulier du casque, avec une autre pièce de traitement des données, en particulier via Bluetooth, rayonnement infrarouge et/ou réseau wi-fi.

10. Dispositif selon l'une quelconque des revendications précédentes, **caractérisé en ce que**
ladite au moins une unité de capteur est présentée séparément dudit au moins un élément en forme d'entonnoir ou ladite au moins une unité de capteur et ledit au moins un élément en forme d'entonnoir peuvent être ou sont connectés de manière amovible l'une à l'autre.

11. Dispositif selon l'une quelconque des revendications précédentes, **caractérisé en ce que**
ledit au moins un quatrième capteur pour la détermination d'une fréquence cardiaque de l'utilisateur peut être ou est disposé dans ladite au moins une unité de capteur ou séparément de celle-ci, en particulier peut être ou est disposé sur le corps de l'utilisateur, ledit au moins un quatrième capteur étant en particulier prévu sous la forme d'un appareil de mesure de fréquence cardiaque, en particulier sans fil, et/ou d'un oxymètre de pouls.

12. Utilisation d'un dispositif selon l'une des revendications 1 à 11 pour une analyse de gaz respiratoire et/ou pour la détermination de données de mesure pour la commande ou la régulation d'au moins une pièce de résistance pilotable ou réglable d'une unité d'entraînement et/ou de rééducation.
